# FASCICULE DE BREVET EUROPEEN

(11) **EP 0 592 283 B1**
(45) Date de publication et mention de la délivrance du brevet: **23.04.1997**
(21) Numéro de dépôt: 93402386.2
(22) Date de dépôt: 30.09.1993
(51) Int. Cl.: C08F 2/38, C07C 333/20, C08F 8/00

(54) **Polymères téléchéliques hydroxylés, leur procédé de fabrication et leurs utilisations**
Hydroxylierte Telechele Polymere, Verfahren zu deren Herstellung und deren Verwendung
Hydroxylated telechelic polymers, process for their preparation and their use

(30) Priorité: 06.10.1992 FR 9211845
(43) Date de publication de la demande: 13.04.1994
(73) Titulaire: ELF ATOCHEM S.A., 92800 Puteaux (FR)
(72) Inventeur: Sarraf, Tarek, F-64140 Lons (FR); Catala, Jean-Marie, F-67450 Mundolsheim (FR)
(74) Mandataire: Chaillot, Geneviève

(56) Documents cités:
- EP-A- 0 237 792
- EP-A- 0 338 918

## Description

La présente invention porte sur des polymères téléchéliques hydroxylés à base de monomères à insaturation éthylénique, polymérisés par voie radicalaire en présence de disulfures de thiurame fonctionnels comme agents ayant la triple fonction d'initiateur, d'agent de transfert de chaîne et d'agent de terminaison, de tels agents étant couramment désignés par l'abréviation "iniferters". La présente invention porte également sur un procédé de fabrication de ces polymères téléchéliques hydroxylés, ainsi que sur leurs utilisations.

Les polymères téléchéliques de ce type, qui englobent les homopolymères et les copolymères statistiques, sont recherchés notamment parce qu'ils peuvent entrer dans la composition de copolymères à propriétés spécifiques en raison du fait qu'ils portent des fonctions particulières. Des disulfures de thiurame fonctionnels sont proposés dans le brevet européen EP-B-0 237 792, à titre d'iniferters, les groupements fonctionnels envisagés comprenant, entre autres, les fonctions hydroxyle.

La synthèse de ces polymères téléchéliques (p) portant des fonctions terminales OH en présence de ces iniferters (i), telle qu'elle est décrite dans le brevet européen précité, peut être schématisée comme suit : 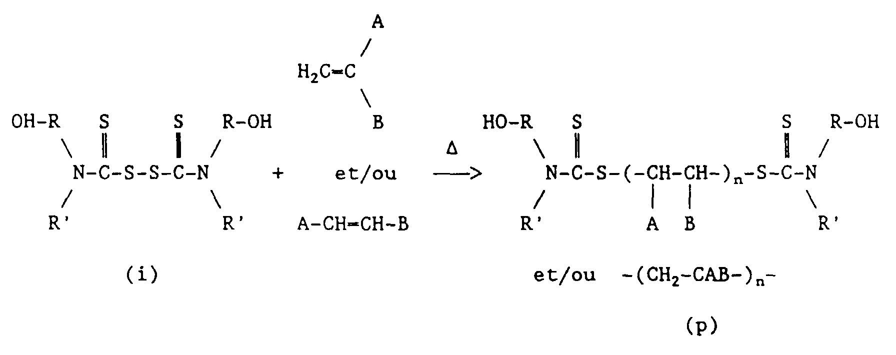 dans lequel :
- A présente diverses significations dont H, CH₃, -COOR^{O}, -CH₂-COOR^{O} avec R^{O} = alkyle ;
- B présente diverses significations dont -COOR^{O}, -CH=CH₂, avec R^{O} = alkyle ;
- R représente alkylène ou cycloalkylène ;
- R' représente alkyle ou cycloalkyle ; et
- n représente le nombre de motifs issus du (ou des) monomère(s) vinylique(s) et/ou diénique(s) mis en jeu.

En règle générale, cette réaction de polymérisation est conduite en masse ou en présence d'un solvant. La température de polymérisation est généralement comprise entre 70 et 150°C.

Dans le cas où R représente -(CH₂)ₙ- avec n = 2 ou 3, il a été constaté qu'il se produit, dans les conditions réactionnelles ci-dessus, une réaction de cyclisation et de formation de produits de décomposition selon le schéma indiqué ci-dessous pour n = 2 :

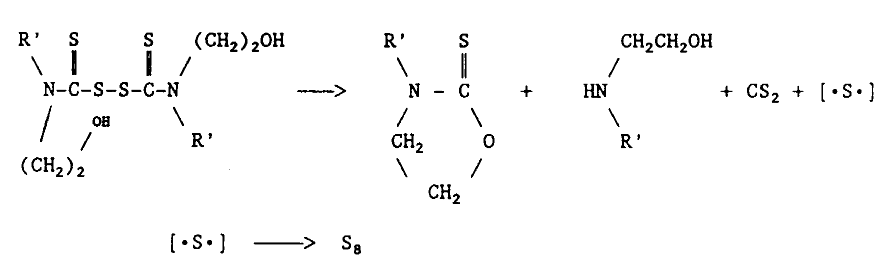

La conséquence de cette décomposition est la formation d'un polymère non téléchélique de masse moléculaire moyenne plus élevée que celle prévue et avec un rendement très faible.

Autrement dit, cette sous-famille des polymères téléchéliques (p) envisagée dans le brevet européen EP-B-O 237 792 ne semble pas avoir été préparée, ce document étant muet en ce qui concerne le problème posé ci-dessus, aussi bien dans l'exposé général de l'invention qui y est décrite que dans ses exemples. Les iniferters mis en oeuvre dans ces derniers ne donnent pas lieu à la réaction de cyclisation et conduisent donc, pour leur part, aux polymères téléchéliques attendus.

La Société déposante a recherché une solution à ce problème, afin de pouvoir proposer les polymères téléchéliques hydroxylés que l'on n'a pas encore réussi à obtenir à l'heure actuelle.

A cet effet, il est proposé, selon l'invention, pour s'affranchir du problème précité, de protéger les fonctions OH des iniferters considérés et, de façon surprenante, les essais qui ont été conduits avec les iniferters protégés ont montré l'absence totale de produits de décomposition et l'obtention des polymères téléchéliques visés avec un bon rendement.

La présente invention a donc d'abord pour objet les polymères téléchéliques hydroxylés, consistant en homopolymères ou copolymères statistiques, qui sont obtenus par voie radicalaire à partir d'au moins un monomère à insaturation éthylénique M choisi parmi les monomères vinyliques et diéniques, et qui sont terminés en α et ω par des groupements hydroxylés représentés par la formule 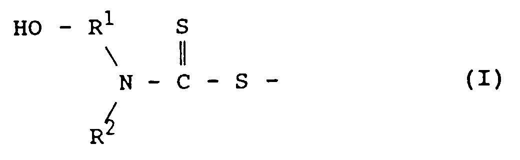 dans laquelle :
- R¹ représente un groupe alkylène en C₂-C₃ ; et
- R² représente un groupe alkyle en C₁-C₁₀, linéaire ou ramifié, ou cycloalkyle ou aryle ou aralkyle.

Les monomères vinyliques qui peuvent être mis en oeuvre selon la présente invention, peuvent être classés en monomères méthacryliques, acryliques et vinylaromatiques.

A titre d'exemples de monomères méthacryliques, on peut citer les méthacrylates d'alkyle dont le radical alkyle, le cas échéant substitué, par exemple par au moins un atome d'halogène comme le chlore ou le fluor et/ou au moins un groupe hydroxyle, contient de 1 à 18 atomes de carbone, comme les méthacrylates de méthyle, d'éthyle, de 2,2,2-trifluoroéthyle, de n-propyle, d'isopropyle, de n-butyle, de sec.-butyle, de tert.-butyle, de n-amyle, d'i-amyle, de n-hexyle, d'éthyl-2 hexyle, de cyclohexyle, d'octyle, d'i-octyle, de décyle, d'hydroxyalkyle inférieurs comme de β-hydroxyéthyle, d'hydroxypropyle, d'hydroxybutyle ; le méthacrylate de glycidyle ; le méthacrylate de norbornyle ; le méthacrylate d'isobornyle ; le méthacrylonitrile ; les dialkylméthacrylamides ; l'acide méthacrylique et les mélanges de ces monomères.

A titre d'exemples de monomères acryliques, on peut citer les acrylates d'alkyle primaire, secondaire ou tertiaire dont le groupe alkyle, le cas échéant substitué par exemple, par au moins un atome d'halogène, tel que chlore ou fluor, et/ou au moins un groupe hydroxyle, après protection de ce groupe hydroxyle, contient 1 à 18 atomes de carbone, en mentionnant plus particulièrement, les acrylates d'éthyle, de propyle, d'isopropyle, de butyle, d'isobutyle, d'hexyle, de tertiobutyle, d'éthyl-2 hexyle, de nonyle, de lauryle, de stéaryle, de cyclohexyle, d'isodécyle. On pourrait également mettre en oeuvre l'acrylate de phényle, l'acrylate de norbornyle, l'acrylate d'isobornyle, l'acrylate de cyanéthyle, les acrylates d'hydroxyalkyle inférieurs, et des acrylates d'alkylthioalkyle ou d'alcoxyalkyle, notamment les acrylates de méthoxy- et éthoxyéthyle, et également l'acrylonitrile, l'acide acrylique et les dialkylacrylamides.

A titre d'exemples de monomères vinylaromatiques, on peut citer le styrène ; les styrènes substitués comme, par exemple, l'alphaméthyl-styrène, le méthyl-4 styrène, le méthyl-3 styrène, le méthoxy-4 styrène, l'hydroxyméthyl-2 styrène, l'éthyl-4 styrène, l'éthoxy-4 styrène, le diméthyl-3,4 styrène, le chloro-2 styrène, le chloro-3 styrène, le chloro-4 méthyl-3 styrène, le tert.-butyl-3 styrène, le dichloro-2,4 styrène et le dichloro-2,6 styrène le vinyltoluène ; le vinyl-l naphtalène ; et les mélanges de ces monomères.

On peut également citer comme monomères vinyliques, l'anhydride maléique, les maléimides substitués par des groupes alkyle ou aryle, la vinyl-2 pyridine et la vinyl-4 pyridine.

Comme monomères diéniques qui peuvent être mis en oeuvre selon l'invention, on peut mentionner, entre autres, de façon plus détaillée, le butadiène, l'isoprène, le 1,3-pentadiène, le 1,4-pentadiène, le 1,4-hexadiène, le 1,5-hexadiène, le 1,9-décadiène, le 5-méthylène-2-norbornène, le 5-vinyl-2 norbornène, les 2-alkyl-2,5-norbornadiènes, le 5-éthylidène-2-norbornène, le 5-(2-propényl)-2-norbornène, le 5-(5-hexényl)-2-norbornène, le 1,5-cyclooctadiène, le bicyclo[2,2,2]octa-2,5-diène, le cyclopentadiène, le 4,7,8,9-tétrahydroindène et l'isopropylidène tétrahydroindène.

Parmi les groupes alkyle entrant dans la définition de R², on peut citer plus particulièrement les groupes alkyle en C₁-C₆, linéaires ou ramifiés ; parmi les groupes cycloalkyle, le groupe cyclohexyle ; et parmi les groupes aralkyle, le groupe benzyle.

Les polymères téléchéliques hydroxylés selon l'invention présentent en outre une masse moléculaire moyenne en nombre généralement comprise entre 1000 et 5000, et les autres caractéristiques suivantes :
Indice de polydispersité = entre 2 et 3
Taux de fonctionnalité = 2

La présente invention porte également sur un procédé de préparation de ces polymères téléchéliques hydroxylés, caractérisé par le fait qu'on conduit une homopolymérisation ou copolymérisation statistique, par voie radicalaire, d'au moins un monomère à insaturation éthylénique M choisi parmi les monomères vinyliques et diéniques, en présence d'un agent iniferter ayant la triple fonction d'initiateur, d'agent de transfert de chaîne et d'agent de terminaison de chaîne, consistant en le disulfure de thiurame de formule : 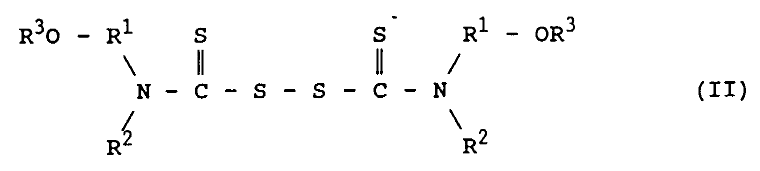 dans laquelle :
- R¹ et R² ont les mêmes significations que celles indiquées ci-dessus ; et
- R³ représente un groupe protecteur d'hydroxyle ;
puis que l'on effectue une réaction de déprotection du groupe OH porté par R¹, pour obtenir un polymère téléchélique présentant des groupements terminaux hydroxylés en α et ω de formule : 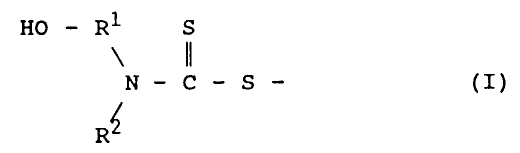 dans laquelle R¹ et R² sont tels que définis ci-dessus.

On utilise généralement une quantité d'iniferter de la formule (II) comprise entre 15 et 25% en poids par rapport au poids du (ou des) monomère(s) M introduits.

Parmi les iniferters de formule (II) à groupes OH protégés que l'on peut utiliser, on peut citer ceux dans lesquels R³ est choisi parmi les groupes : 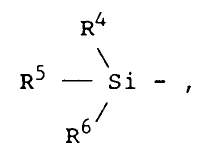 R⁴, R⁵ et R⁶ représentant chacun indépendamment un groupe alkyle en C₁-C₆, et les groupes 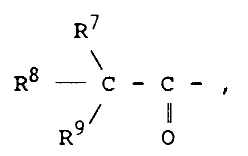 R⁷, R⁸ et R⁹ représentant chacun indépendamment hydrogène ou un groupe alkyle en C₁-C₆.

On les obtient en faisant réagir l'iniferter non protégé correspondant avec un composé de formule R³-D, D représentant halogène, tel que chlore. On peut citer ainsi la réaction d'un iniferter non protégé avec le chlorure de triméthylsilane en milieu solvant anhydre comme le tétrahydrofuranne, l'éther, etc., en présence d'une amine tertiaire, comme la triéthylamine, avec un léger excès stoechiométrique du chlorure de triméthylsilane. L'amine tertiaire a pour rôle de bloquer HCl sous forme de sel. On peut également citer la réaction d'un iniferter non protégé avec 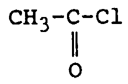 dans les mêmes conditions que celles décrites précédemment.

En ce qui concerne les conditions pour la polymérisation selon l'invention, on peut mentionner une température de polymérisation généralement comprise entre 50 et 160°C, notamment, entre 70 et 150°C et plus particulièrement entre 80 et 110°C, et une durée de polymérisation généralement comprise entre 1 et 24 heures. Par ailleurs, on introduit généralement l'iniferter de formule (II) en début de polymérisation. Celle-ci est par ailleurs conduite en masse, en solution ou en suspension, dans un milieu solvant tel que le diméthylsulfoxyde, le toluène, l'acétate de butyle, le tétrahydrofuranne.

L'étape de déprotection est conduite par hydrolyse en milieu acide dans le cas où R³ représente R⁴,R⁵,R⁶-Si- et par hydrolyse en milieu acide ou basique dans le cas où R³ représente R⁷,R⁸,

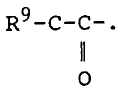

La présente invention porte également sur l'utilisation des polymères téléchéliques hydroxylés qui viennent d'être décrits, comme précurseurs dans la synthèse de hauts-polymères linéaires ou réticulés ; comme constituants de solutions, émulsions, dispersions ou suspensions aqueuses ; pour la préparation de solutions, émulsions, dispersions et suspensions aqueuses de hauts-polymères linéaires ou réticulés issus desdits polymères téléchéliques hydroxylés ; comme précurseurs réactifs de liants hauts-polymères linéaires ou réticulés dans des systèmes aqueux d'agents de peinture ; dans des systèmes aqueux pour le dépôt électrophorétique de revêtements ; après dépôt électrophorétique dans des systèmes aqueux, comme précurseurs réactifs de revêtements en hauts-polymères linéaires ou réticulés ; dans la fabrication d'élastomères ; et pour modifier des surfaces.

Pour mieux illustrer l'objet de la présente invention, on va en décrire ci-après plusieurs exemples de réalisation. Dans ces exemples, le monomère étudié est le méthacrylate de méthyle, indiqué par l'abréviation MAM.

### Exemples comparatifs 1 à 6 :

Polymérisation en masse du MAM en présence d'iniferters hydroxylés non protégés. Rapport constant [MAM] /[Iniferter]

### Mode opératoire général

Dans un réacteur d'une capacité de 1 litre, on a introduit 101 g (1 mole) de MAM et x grammes (0,32 mole) de l'iniferter étudié. On porte le mélange à une température T, pendant t heures. Après la réaction, on effectue une analyse du mélange par chromatographie sur gel perméable ou par chromatographie en phase vapeur couplée avec spectre de masse, laquelle montre la présence de produits de décomposition. Après évaporation sous vide, on obtient y grammes de polymère (Rendement : R%).

Dans le Tableau récapitulatif ci-après, sont indiquées les conditions particulières dans lesquelles ont été conduits ces Exemples comparatifs 1 à 6.

### Exemple 7 :

Préparation d'un iniferter de formule (II) dans laquelle :

R³ = (CH₃)₃Si-

R¹ = -CH₂-CH₂- ;

et

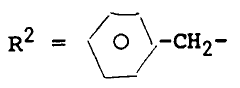

Dans un tricol placé sous azote, on a introduit 250 ml de tétrahydrofuranne anhydre, ainsi que 45,2 g (0,1 mole) de l'iniferter utilisé dans les Exemples comparatifs 3 à 6. Après addition de 21,2 g (0,21 mole) de triéthylamine, on a ajouté, goutte à goutte, sous agitation 22,8 g (0,21 mole) de chlorure de triméthylsilane. Après 12 heures de réaction, le milieu a été évaporé sous vide. Le composé silylé a été séparé du chlorhydrate de triéthylamine par solubilisation dans le benzène. Après filtration et évaporation, on a récupéré 59,6 g (0,1 mole) de l'iniferter de l'intitulé. Rendement de la réaction : 100%.

### Exemples 8 à 11 :

Polymérisation en masse du MAM en présence de l'iniferter de l'Exemple 7.

### Mode opératoire général

Dans un réacteur de 1 litre, on a introduit 101 g (1 mole) de MAM et 19,1 g (0,032 mole) de l'iniferter de l'Exemple 7. On a porté le tout à T°C pendant t heures. Après la réaction, l'analyse du mélange par chromatographie sur gel perméable a montré l'absence de produits de décomposition. Après évaporation sous vide, on a obtenu un polymère avec un rendement de R%. L'hydrolyse de ce produit en milieu acide a conduit au polymère téléchélique attendu.

On a donc pu montrer que le groupement protecteur n'est pas modifié par la température et que les réactions secondaires n'ont pas lieu. Ceci permet d'effectuer les réactions de polymérisation radicalaire dans un temps relativement court, en ayant des rendements élevés.

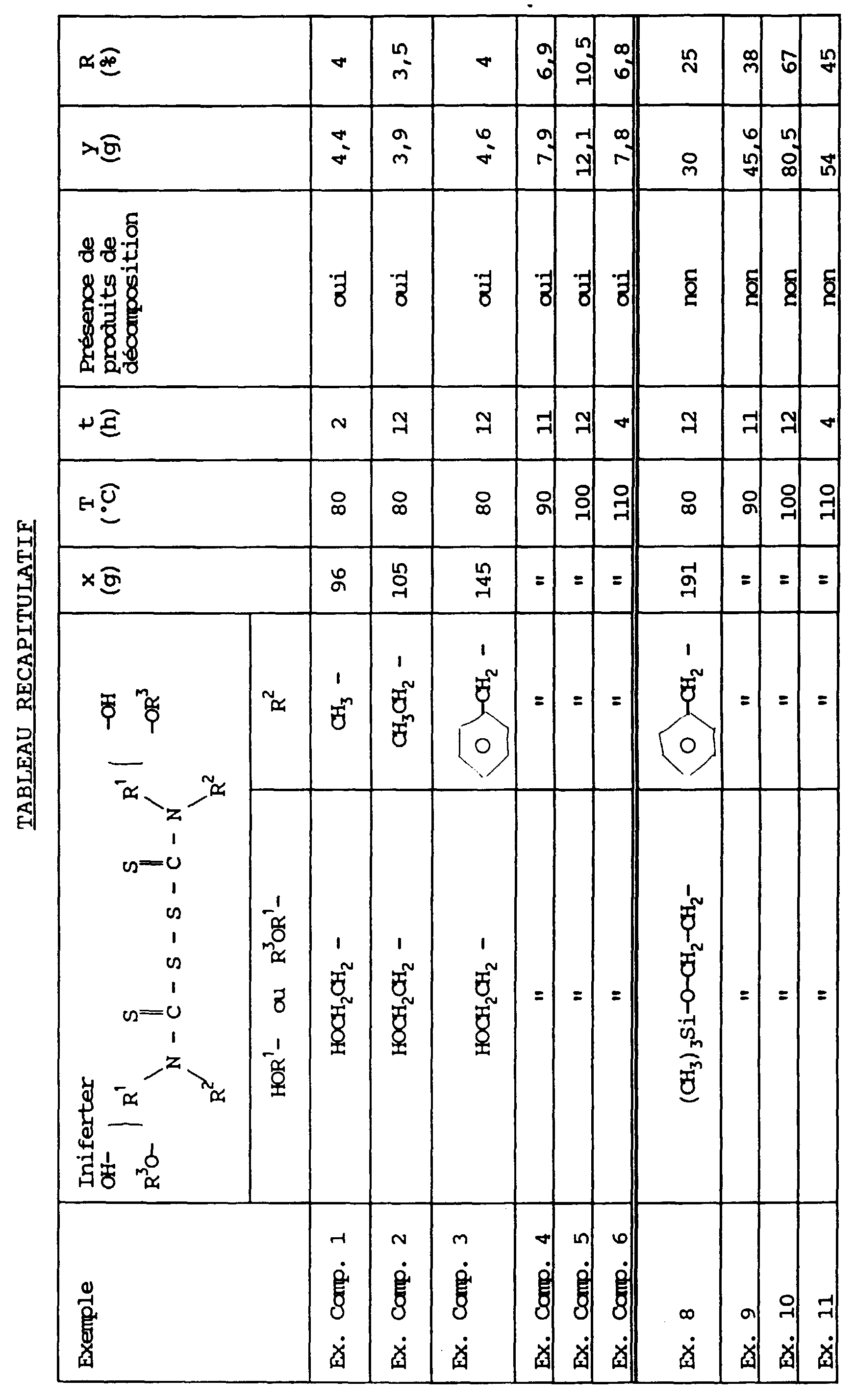

## Revendications

1. Polymères téléchéliques hydroxylés, consistant en homopolymères ou copolymères statistiques, qui sont obtenus par voie radicalaire à partir d'au moins un monomère à insaturation éthylénique M choisi parmi les monomères vinyliques et diéniques, et qui sont terminés en α et ω par des groupements hydroxylés représentés par la formule : 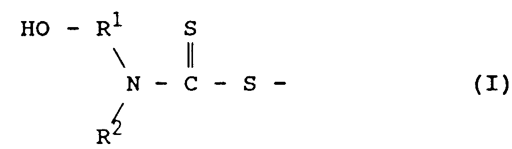 dans laquelle :
- R¹ représente un groupe alkylène en C₂-C₃ ; et
- R² représente un groupe alkyle en C₁-C₁₀, linéaire ou ramifié, ou cycloalkyle ou aryle ou aralkyle.

2. Procédé de préparation des polymères téléchéliques hydroxylés tels que définis à la revendication 1, caractérisé par le fait qu'on conduit une homopolymérisation ou copolymérisation statistique, par voie radicalaire, d'au moins un monomère à insaturation éthylénique M choisi parmi les monomères vinyliques et diéniques, en présence d'un agent iniferter ayant la triple fonction d'initiateur, d'agent de transfert de chaîne et d'agent de terminaison de chaîne, consistant en le disulfure de thiurame de formule : 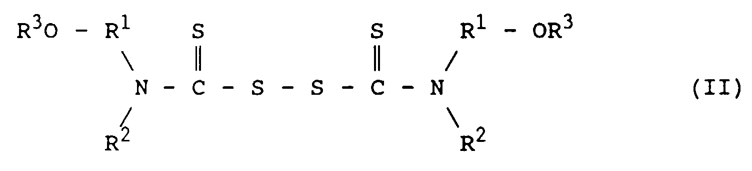 dans laquelle :
- R¹ et R² ont les mêmes significations que celles indiquées à la revendication 1 ; et
- R³ représente un groupe protecteur d'hydroxyle ;
puis que l'on effectue une réaction de déprotection du groupe OH porté par R¹, pour obtenir un polymère téléchélique présentant des groupements terminaux hydroxylés en α et ω de formule : 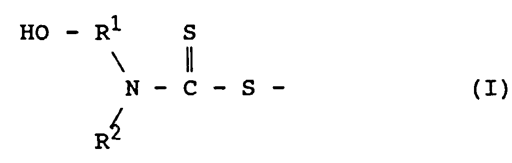 dans laquelle R¹ et R² sont tels que définis à la revendication 1.

3. Procédé selon la revendication 2, caractérisé par le fait que l'on utilise une quantité d'iniferter de la formule (II) comprise entre 15 et 25 % en poids par rapport au poids du (ou des) monomère(s) M introduits.

4. Procédé selon l'une des revendications 2 et 3, caractérisé par le fait qu'on utilise un iniferter de la formule (II) dans laquelle le groupe protecteur R³ est choisi parmi les groupes : 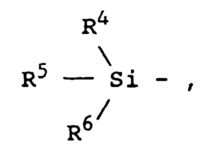 R⁴, R⁵ et R⁶ représentant chacun indépendamment un groupe alkyle en C₁-C₆, et les groupes 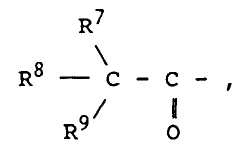 R⁷, R⁸ et R⁹ représentant chacun indépendamment hydrogène ou un groupe alkyle en C₁-C₆.

5. Procédé selon l'une des revendications 2 à 4, caractérisé par le fait que l'on conduit la polymérisation en masse, en solution ou en suspension, à une température comprise entre 50 et 160°C et pendant un laps de temps compris entre 1 et 24 heures.

6. Procédé selon l'une des revendications 2 à 5, caractérisé par le fait que l'on introduit l'iniferter en début de polymérisation.

7. Procédé selon l'une des revendications 2 à 6, caractérisé par le fait qu'on conduit l'étape de déprotection par hydrolyse en milieu acide dans le cas où R³ représente 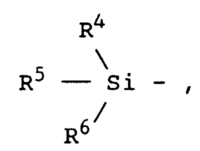 R⁴, R⁵ et R⁶ étant tels que définis à la revendication 4, et par hydrolyse en milieu acide ou basique dans le cas où R³ représente 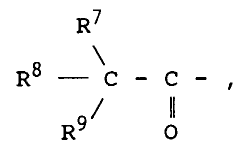 R⁷, R⁸ et R⁹ étant tels que définis à la revendication 4.

8. A titre de produit utile dans la synthèse des polymères téléchéliques hydroxylés tels que définis à la revendication 1, le disulfure de thiurame représenté par la formule (II) telle que définie à la revendication 2.

9. Utilisation des polymères téléchéliques tels que définis à la revendication 1, comme précurseurs dans la synthèse de hauts-polymères linéaires ou réticulés ; comme constituants de solutions, émulsions, dispersions ou suspensions aqueuses ; pour la préparation de solutions, émulsions, dispersions et suspensions aqueuses de hauts-polymères linéaires ou réticulés issus desdits polymères téléchéliques hydroxylés ; comme précurseurs réactifs de liants hauts-polymères linéaires ou réticulés dans des systèmes aqueux d'agents de peinture ; dans des systèmes aqueux pour le dépôt électrophorétique de revêtements ; après dépôt électrophorétique dans des systèmes aqueux, comme précurseurs réactifs de revêtements en hauts-polymères linéaires ou réticulés ; dans la fabrication d'élastomères ; et pour modifier des surfaces.

## Patentansprüche

1. Hydroxylierte telechele Polymere, bestehend aus statistischen Homopolymeren oder Copolymeren, die auf radikalischem Weg ausgehend von mindestens einem ethylenisch ungesättigten Monomer M erhalten sind, das ausgewählt ist aus Vinyl- und Dienmonomeren, und die in α- und ω-Position endständige hydroxylierte Gruppen aufweisen, die durch die Formel (I) wiedergegeben werden 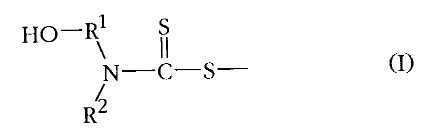 in der:
- R¹ eine C₂-C₃-Alkylengruppe darstellt;
- R² eine lineare oder verzweigte C₁-C₁₀-Alkylgruppe, eine Cycloalkylgruppe, eine Arylgruppe oder eine Aralkylgruppe darstellt.

2. Verfahren zur Herstellung hydroxylierter telecheler Polymere gemäß Anspruch 1, dadurch gekennzeichnet, daß man auf radikalischem Weg eine statistische Homopolymerisation oder Copolymerisation mindestens eines ethylenisch ungesättigten Monomers M, ausgewählt aus Vinyl- oder Dienmonomeren, in Gegenwart eines Iniferters mit dreifacher Funktion, nämlich der eines Starters, der eines Kettenübertragungsmittels und der eines Kettenabbruchmittels, durchführt, der aus Thiuramdisulfid der Formel (II) 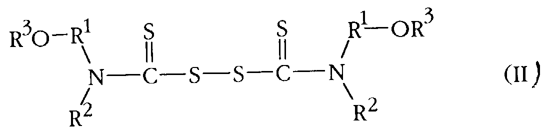 besteht, in der:
- R¹ und R² die in Anspruch 1 angegebene Bedeutung haben; und
- R³ eine Schutzgruppe für das Hydroxyl darstellt;
man dann die an R¹ befindliche OH-Schutzgruppe entfernt, um ein telecheles Polymer zu enthalten, das in α- und ω-Position endständige hydroxylierte Gruppen der Formel 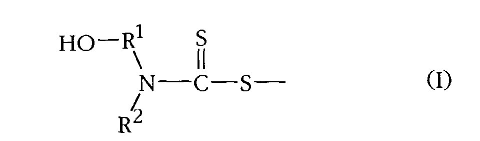 aufweist, in der R¹ und R² die in Anspruch 1 angegebene Bedeutung haben.

3. Verfahren nach Anspruch 2, dadurch gekennzeichnet, daß man eine Menge des Iniferters der Formel (II) zwischen 15 und 25 Gew.-% verwendet, bezogen auf das Gewicht des oder der eingebrachten Monomere M.

4. Verfahren nach Anspruch 2 oder 3, dadurch gekennzeichnet, daß man einen Iniferter der Formel (II) verwendet, in der die Schutzgruppe R³ ausgewählt ist aus Gruppen der Formel 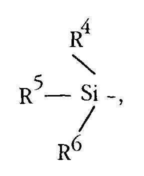 wobei R⁴, R⁵ und R⁶ jeweils, unabhängig voneinander, eine C₁-C₆-Alkylgruppe darstellen, und
aus Gruppen der Formel 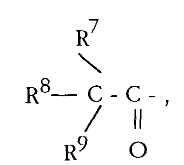 wobei R⁷, R⁸ und R⁹ jeweils, unabhängig voneinander, Wasserstoff oder eine C₁-C₆-Alkylgruppe bezeichnen.

5. Verfahren nach einem der Ansprüche 2 bis 4, dadurch gekennzeichnet, daß man die Polymerisation in Masse, in Lösung und in Suspension bei einer Temperatur zwischen 50 und 160 °C und während einer Zeitdauer zwischen 1 und 24 Stunden durchführt.

6. Verfahren nach einem der Ansprüche 2 bis 5, dadurch gekennzeichnet, daß man den Iniferter zu Beginn der Polymerisation einbringt.

7. Verfahren nach einem der Ansprüche 2 bis 6, dadurch gekennzeichnet, daß man den Schritt der Schutzgruppenentfernung mittels Hydrolyse im sauren Milieu durchführt, falls R³ 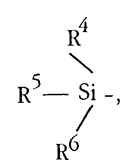 darstellt, wobei R⁴, R⁵ und R⁶ die in Anspruch 4 angegebene Bedeutung haben, und mittels Hydrolyse im sauren oder basischen Milieu durchführt, falls R³ eine Gruppe 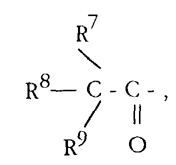 darstellt, wobei R⁷, R⁸ und R⁹ die in Anspruch 4 angegebene Bedeutung haben.

8. Thiuramdisulfid gemäß Formel (II), wie in Anspruch 2 definiert, als nützliches Produkt bei der Synthese hydroxylierter telecheler Polymere gemäß Anspruch 1.

9. Verwendung telecheler Polymere gemäß Anspruch 1 als Vorläufer in der Synthese von linearen oder vernetzten Hochpolymeren; als Bestandteile von wäßrigen Lösungen, Emulsionen, Dispersionen oder Suspensionen; zur Herstellung von wäßrigen Lösungen, Emulsionen, Dispersionen und Suspensionen von linearen oder vernetzten Hochpolymeren, die aus den hydroxylierten telechelen Polymeren hervorgehen; als reaktive Vorläufer von linearen oder vernetzten Hochpolymerenbindemitteln in wäßrigen Systemen von Anstrichmitteln; in wäßrigen Systemen zur elektrophoretischen Abscheidung von Beschichtungen; nach elektrophoretischer Abscheidung in wäßrigen Systemen als reaktive Vorläufer von Beschichtungen aus linearen oder vernetzten Hochpolymeren; bei der Herstellung von Elastomeren; und zur Veränderung von Oberflächen.

## Claims

1. Hydroxylated telechelic polymers, consisting of homopolymers or statistical copolymers, which are obtained by the radical route from at least one monomer containing ethylenic unsaturation M chosen from vinyl and diene monomers and which are terminated at the α and ω positions by hydroxylated groups represented by the formula: 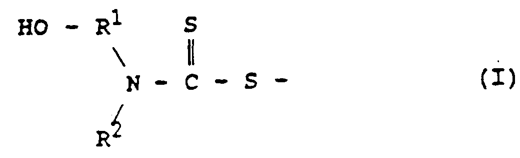 in which:
- R¹ represents a C₂-C₃ alkylene group; and
- R² represents a linear or branched C₁-C₁₀ alkyl or cycloalkyl or aryl or aralkyl group.

2. Process for the preparation of the hydroxylated telechelic polymers as defined in Claim 1, characterized in that a homopolymerization or statistical copolymerization is carried out, by the radical route, of at least one monomer containing ethylenic unsaturation M chosen from vinyl and diene monomers, in the presence of an iniferter agent having the three-fold function of initiator, of chain transfer agent and of chain termination agent, comprising the thiuram disulphide of formula: 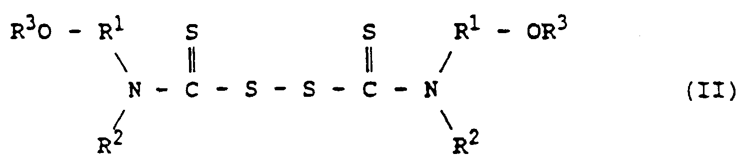 in which:
- R¹ and R² have the same meanings as those indicated in Claim 1; and
- R³ represents a protective group for hydroxyl;
and in that a deprotection reaction of the OH group carried by R¹ is then carried out, in order to obtain a telechelic polymer exhibiting hydroxylated terminal groups at the α and ω positions of formula: 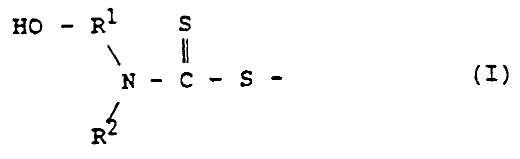 in which R¹ and R² are as defined in Claim 1.

3. Process according to Claim 2, characterized in that use is made of an amount of iniferter of the formula (II) of between 15 and 25% by weight with respect to the weight of the monomer(s) M introduced.

4. Process according to either of Claims 2 and 3, characterized in that use is made of an iniferter of the formula (II) in which the protective group R³ is chosen from the groups: 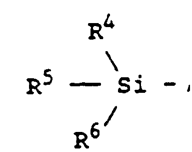 R⁴, R⁵ and R⁶ each independently representing a C₁-C₆ alkyl group, and the groups 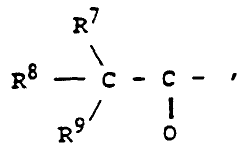 R⁷, R⁸ and R⁹ each independently representing hydrogen or a C₁-C₆ alkyl group.

5. Process accdrding to one of Claims 2 to 4, characterized in that the polymerization is carried out in bulk, in solution or in suspension, at a temperature of between 50 and 160°C and for a period of time of between 1 and 24 hours.

6. Process according to one of Claims 2 to 5, characterized in that the iniferter is introduced at the beginning of polymerization.

7. Process according to one of Claims 2 to 6, characterized in that the deprotection stage is carried out by hydrolysis in acidic medium in the case where R³ represents 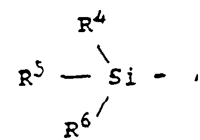 R⁴, R⁵ and R⁶ being as defined in Claim 4, and by hydrolysis in acidic or basic medium in the case where R³ represents 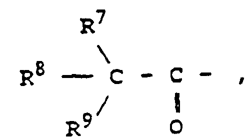 R⁷, R⁸ and R⁹ being as defined in Claim 4.

8. As product which is useful in the synthesis of the hydroxylated telechelic polymers as defined in Claim 1, the thiuram disulphide represented by the formula (II) as defined in Claim 2.

9. Use of the telechelic polymers as defined in Claim 1, as precursors in the synthesis of linear or crosslinked high polymers; as constituents of aqueous solutions, emulsions, dispersions or suspensions; for the preparation of aqueous solutions, emulsions, dispersions and suspensions of linear or crosslinked high polymers resulting from the said hydroxylated telechelic polymers; as reactive precursors of linear or crosslinked high polymer binders in aqueous coating systems; in aqueous systems for the electrophoretic deposition of coatings; after electrophoretic deposition in aqueous systems, as reactive precursors of linear or crosslinked high polymer coatings; in the manufacture of elastomers; and for modifying surfaces.
